Europäisches Patentamt

European Patent Office

Office européen des brevets

(11) Publication number: **0 222 967**
A1

(12) **EUROPEAN PATENT APPLICATION**

(21) Application number: 86103234.0

(22) Date of filing: 11.03.86

(51) Int. Cl.⁴: **A61K 31/46** , A23L 1/308

(30) Priority: 08.11.85 US 796202

(43) Date of publication of application:
27.05.87 Bulletin 87/22

(84) Designated Contracting States:
AT BE CH DE FR GB IT LI NL

(71) Applicant: Coyne, Martin J., Dr. (M.D.)
9104 St. Ives
Los Angeles California 90069(US)

(72) Inventor: Coyne, Martin J., Dr. (M.D.)
9104 St. Ives
Los Angeles California 90069(US)

(74) Representative: LOUIS, PÖHLAU, LOHRENTZ &
SEGETH
Kesslerplatz 1 P.B. 3055
D-8500 Nürnberg(DE)

(54) Composition for controlling weight and its use.

(57) A composition for controlling weight and particularly for reducing weight by controlling gastric emptying as well as its use for manufacture.

The composition comprises a combination of an anticholinergic agent along with an ingestible fiber and preferably that viscous fiber containing galactose and mannose, in the form of a gum and of the type offered under the commercial name "guar". The composition includes a major amount of the viscous fiber and a minor amount of the anticholinergic agent although each provides essentially the same bio-availability and the same biological activity. The combination of the anticholinergic agent and the Guar gum presents a synergistic effect which would not be expected from a combination of the two components. In addition, the composition can be used in combination with a specific dietary plan to further provide for weight reduction.

## COMPOSITION FOR CONTROLLING WEIGHT AND ITS USE

This invention relates in general to a composition and a method for manufacture of a substance reducing weight by reducing gastric emptying and which composition is comprised of an ingestible fiber as well as an anticholinergic agent.

It has been well known that a control of gastric emptying will provide some control over the amount of food intake of an individual and hence, this will provide some control over the weight of an individual. Indeed, in recent years, there has been a much greater understanding of the effects of gastric motility on the effects of weight of an individual. The significance of the various pathophysiologic and diagnostic advances in the effects of gastric emptying is presented in Gastroenterology, 1984-1986 "The Physiology and Pathphysiology of Gastric Emptying in Humans" by Howard Minami and Richard W. McCallum.

Studies of overweight conditions as a function of gastric emptying has also been reported in Gastroeneterology, Volume 84, No. 4, April, 1983 "Obesity and Gastric Emptying". Also, the effects of gastric emptying and obesity have been reported in Gastroeneterology, 1983-1984; 747-51 "Gastric Emptying and Obesity" by Richard A. Wright et al.

Patients with exogenous obesity are generally known to have rapid gastric emptying. This condition has been known and reported in Gastroenterology, 1983-84; 747-51 "Gastric Emptying and Obesity" by R. A. Wright et al. The altered physiology as a result of rapid gastric emptying may well contribute to the feeling of hunger in these patients because distension of the fundus is necessary to achieve satiety. When gastric emptying is slowed under pathiophysiological conditions, the appetite is decreased and this will result in diminshed food in-take and hence weight loss.

It is known that gastric emptying can occur pathologically in various metabolic conditions such as diabetes mellitus and with pyloric obstruction secondary to tumor involvement or ulcer disease.

Surgical operations have been designed to take advantage of the effect of slow gastric emptying on weight control. Surgical gastroplasty appears to be a moderately successful procedure which is currently available for weight reduction. However, this technique suffers from the attendant disadvantages that surgery is not only expensive and carries post operative risk, but it also has demonstrated a 50% to 70% failure rate after twenty four months.

Various non-surgical means have been employed to slow gastric emptying both for control of the dumping syndrome and for weight control. Pectin and guar gum have been found to be effective in slowing gastric emptying for the purpose of weight control. However, the success rate has varied and the use of these substances has not shown any substantial efficacy. The low success rate may be partially explained by the adaptive relaxation of the fundus, thereby increasing the gastric reservoir capacity of the stomach.

Heretofore, the reduction of gastric emptying was viewed as an abnormal condition as reported in "The Physiology and Patheophysiology of Gastric Emptying in Humans" by Howard Manimi et al, supra.

The fiberous composition, pectin, has also been known to reduce gastric emptying and thus, provide some reduction over the amount of food intake. For example, the effects of sustained pectin ingestion in gastric emptying has been reported in Gastroenterology, 1982, 83:812-17 "Sustained Pectin Ingestion Delays Gastric Emptying" by S. E. Schwartz et al.

The use of guar gum has been reported as being beneficial in reducing total cholesterol in hypocholesterolemic patients without affecting the HDL-cholesterol. Human Nutrition; Clinical Nutrition (1983) "A Double-Blind Evaluation of Guar Gum In Patients With Dyslipidaemia" by Jaakko Tuomilehto, et al.

The use of guar gum in preventing dumping syndrom, which is a well recognized complication of gastric resection, is also reported in Journal of Parenteral and Enteral Nutrition, 1983, "Efficacy of Guar Gum In Preventing the Dumping Syndrom" by E. Harju et al. Further, it has also been established that guar gum has an effect on the absorption of digoxin and phenoxymethyl penicillin on human beings as reported in European Journal of Clinical Pharmacology, Spring, 1984, 26:279-281 "Effect of Guar Gum, a Fiber Preparation, On Digoxin and Penicillin Absorption in Man" by R Huupponen, et al.

In all the cases, the use of guar gum was to overcome a physiological disorder, although the effects on gastric emptying were viewed as an undesirable side effect.

Anticholinergic agents have also been known and widely used in pharmacological preparations particularly, for antidepressants and antihistimines. It is also known that anticholinergic agents have some effect on gastric emptying in the malnourished or chronically ill patient, which is viewed as an adverse side effect. In the healthy patient, this effect is almost non-existent.

The present invention resides in the suprising discovery that the combination of an anticholinergic agent as well as an ingestible fiber, such as e.g. a guar gum, provides a synergistic effect in controlling gastric emptying, as hereinafter described.

It is, therefore, one of the primary objects of the present invention to provide a composition which is highly effective in controlling weight by reduction of gastric emptying without any significant undesirable side effects.

It is another object of the present invention to provide a composition of the type stated which comprises a major amount of an ingestible fiber as well as a minor amount of an anticholinergic agent, but which provides essentially the same biologic activity as the major amount of the viscous fiber.

It is a further object of the present invention to provide a composition of the type stated which utilizes an ingestible fiber as well as an anticholinergic agent and which is highly effective in reducing gastric emptying.

It is an additional object of the present invention to provide a method of controlling the weight of an individual by reducing gastric emptying through controlled intake of a composition containing an ingestible fiber and an anticholinergic agent.

It is also a salient object of the present invention to provide a method of reducing weight by ingesting in a controlled regime and amounts a composition of the present invention and without the necessity of engaging in another form of food intake control.

It is a salient object of the present invention to provide a method of reducing weight by injesting a composition of the type stated along with use of a specific dietary plan to further provide for weight reduction.

With the above and other objects in view, my invention resides in the novel composition and method as hereinafter described in more detail.

The present invention is based on the surprising discovery that an anticholinergic agent and an ingestible fiber, such as e.g. a guar gum, provide a highly effective composition for reducing gastric emptying. Several ingestible fibers in combination with the anticholinergic agent have been found to be effective for reducing the gastric emptying time. Some of these fibers, such as guar gum and pectin, as hereinafter described have been known to have the effects of reducing gastric emptying time, as reported above. However, it has been found that there is an actual synergism between the ingestible fiber and the anticholinergic agent such that the control over gastric emptying time is much greater than would have otherwise been anticipated.

One of the more preferred ingestible fibers is that known as guar gum, although other ingestible fibers are operative in accordance with the present invention. The term "ingestible fiber" is deemed to include any of those fibers which are edible and drinkable by an individual in a controlled regime and amount which does not create any substantial adverse side effects.

It has been known that a guar gum has the effects of reducing gastric emptying as reported above. Guar gum has also been used with some efficacy in the control of various other physiological dysfunction. The use of anticholinergic agents are also well known as pharmacological preparations as for example, as antidepresants and antihistimines. However, it was not heretofore recognized that a minor amount of an anticholinergic agent in combination with a major amount of a viscous fiber, such as a guar gum could produce the desired efficacy in reducing gastric emptying.

More specifically, the composition of the present invention is based on about 94.5 % or greater of an ingestible fiber and about 0.0001% to about 5.5% of an anticholinergic agent. The method of the invention utilizes this composition which is administered to individuals at regular intervals, as for example, three times to four times a day. On this basis and without any other control of food intake, the tested subjects showed a substantial decrease in weight as a result of a substantial reduction in gastric emptying.

It has been realized that when gastric emptying is slowed under pathophysiological conditions, appetite is also decreased. This will result in diminished food intake and hence weight loss.

Tests utilizing the composition and the method of the present invention revealed a prolongation of the rate of gastric emptying by at least 50% above a base line measurement on many obese patients who were attempting to use pectin as a means to reduce gastric emptying.

The present invention also provides for the use of a composition of the type stated in combination with a dietary plan in order to provide for further weight reduction. In accordance with this dietary plan, approximately one thousand calories (ca. 4200 joules) of food is designed to be ingested by each patient in a controlled food intake. This food in-take in combination with the composition has been found to be an effec tive means to reduce weight without any serious noticeable side effects to the patients.

The dietary plan generally relies upon a bran input for breakfast, generally in an amount of about eight grams to about twelve grams. The lunch relies primarily upon vegetables, as for example, in a salad form and usually includes about four to eight ounces (115 to 230 grams) of vegetables.

The dinner relies primarily on one portion of meat generally in an amount of about three ounces (90 grams) and one portion of starch, generally in an amount of about four to eight ounces (115 to 230 grams).

The composition of the present invention is also highly effective when used in combination with the presently available high protein-low calorie liquid diets, often referred to as "chemically defined diets". These diets generally rely upon about no more than 700 calories (3000 joules) intake per day over a reasonable period of time and are generally designated to contain the proper amount of amino acids, carbohydrates, vitamins and minerals. When used in combination with the composition of the present invention, very effective rapid lean body mass loss is achieved.

This invention possesses many other advantages and has other purposes which may be made more clearly apparent from a consideration of the forms in which it may be embodied. These forms will now be described in detail for the purposes of illustrating the general principles of the invention; but it is to be understood that such detailed descriptions are not to be taken in a limiting sense.

As indicated previously the present invention resides in the discovery that an anticholinergic agent along with an ingestible fiber provides an effective composition for reducing gastric emptying by increasing the gastric emptying time and hence provides an effective composition for weight control. Essentially any edible or drinkable fiber of the type previously described may be employed. Some of the edible or drinkable fibers which are known to be effective are the polysaccaride containing viscous fibers, such as the galactomannan. Other edible or drinkable fibers which are effective in combination with the anticholinergic agent are gluco-mannan, pectin, bran fibers, such as ceral bran fibers, lignin fibers and vegetable cellulose fibers. While any of the above fibers are effective, the guar gum fiber, as hereinafter described, has been found to one of the most effective of those fibers in combination with the anticholingeric agent.

Guar gum is a gel-forming fiber and more particularly is the ground endosperm of the seeds of cyamopsis tetragonolobus. Chemically, guar gum is a galactomannan having a molecular weight of approximately 220,000. One such commercially available guar gum is offered under the name or mark "Guarem".

Guar gum is known to retard the absorption of other carbohydrates from the intestine. As a result, guar gum reduces insulin secretion and also has a hypoglycaemic effect. In many cases, the guar gum contains both galactose as well as mannose. The guar gum contains not less than about 66% of a large molecule hydrocolloidal polysaccharide which is generally comprised of D-galactose and D-mannose units in the ratio of about 1 to 2 and bound by glycoside linkages.

The guar gum or other similar viscous polysacchride containing fiber is mixed with an anticholinergic agent, hereinafter described in such manner that each have essentially the same bioavailability and the same physiologic effect in reducing gastric emptying. Essentially any known anticholinergic agent can be used in accordance with the present invention.

The anticholinergic agent is known to interrupt the nerve pathways which are stimulated by a cholinergic. Acetyl cholene is the agent which enables transmission of one nerve ending to another in the gastrointestinal tract. The anticholinergic agent will block these nerve endings such that the cholinergic nerve is prohibited from stimulating the emptying of the stomach. The anticholinergic agent is added to the guar gum, or other polysacchride containing viscous fiber, in a wide range, depending upon the physiologic activity of each of the components. With an anticholinergic agent having a high biologic availability, such as scopolamine, only a minor amount is needed, as for example, 0.0002%. Where the anticholinergic agent has a substantially lesser effect, as for example, with diphenhydramine, as much as 3.85% of the polysacride containing viscous fiber may be used. Generally, the amount of anticholinergic agent will range from about 0.0001 % to about 5.5% and more preferably, will range from about 0.0002% to about 3.85%.

Each of the fiber compound and the anticholinergic compound should have a bio-availability of about 50% or more. More preferably, the fiber compound should have a bio-availability of about 80% or greater and the anticholinergic agent should have a bio-availability of about 80% or greater.

Essentially any of the well known anticholinergic compounds are operable with the present invention. However, there are three preferred groups of anticholinergic compounds which have been found to be highly effective in the present invention. The first of these anticholinergic groups is the belladona group and include for example, scopolamine, atropine and hyosamine. The scopolamine is a highly active compound and should be administered in about 0.007 miligrams three to four times per day. The atropine is also a fairly bioactive anticholinergic compound and should be administered in an amount of about 0.0194 milligrams about three to four times per day. The hyosamine should be administered in an amount of about 0.25 to about 1.25 milligrams three to four times per day.

A second group of anticholinergic compounds which is effective in the present invention is the antidepresant medications as for example, anitriptyline and doxepin. The anitriptyline should be administered about 150 milligrams per day, maximum. Moreover, the doxepin should not be administered in an amount greater than about 150 milligrams per day in combination with the viscous fiber compound.

The third group of anticholinergic agents which have been found to be highly effective are the antihistimines and include for example, diphenhydramine often offered under the name or commercial designation "Benadryl", and brompheniramine. The diphenhydramine should be administered in an amount of about 200 milligrams per day along with the viscous fiber and the brompheniramine should be administered in an amount of about 16 milligrams per day along with the viscous fiber compound.

The composition of the present invention is a mixture of the cholinergic agent and the fiber in the amounts as specified. Thus, for example, approximately five to 10 grams of the fiber, such as the guar gum, will be mixed with the selected anticholinergic compound. As an example, 5 to 10 grams of Guar in a mixture with about 0.125 to about 0.25 milligrams of hyoscamine will be mixed and administered to a patient three to four times per day. If desired, the two compounds could be administered separately three to four times per day, although they will be preferably administered as a mixture.

Studies have revealed that the composition of the present invention is far more effective in controlling gastric emptying than is any anticholinergic agent in any normal healthy individual and is far more effective individually than the Guar viscous fiber or any other fiber compound. Tests have also revealed that there is a marked increase in the time of gastric emptying when patients are given the compositions of the present invention without any other active control on the part of the patient. Thus, there is no need for the patient to attempt to overtly control food intake inasmuch as the gastric emptying will automatically satiate the patients apetite and reduce the desire for food intake.

The present invention also provides a method for reducing weight by using a combination of the composition along with a specifc dietary plan. Under this dietary plan, the patient generally takes in about one thousand calories (ca. 4200 joules) per day in a normal diet, but an amount not to exceed 1.500 calories (6300 joules). This diet provides sufficient nutrition for the patient and in combination with the composition of the present invention is generally sufficeint to satisfy the patients desire for food. Hence, the dietary program along with the composition has been sufficient to provide for significant weight reduction.

The dietary program generally relies upon a controlled breakfast, lunch and dinner. The breakfast generally includes a bran type cereal in an amount of about 4 ounces (115 grams). The lunch is based upon vegetables, generally fresh vegetables, preferably in the nature of salad. For example, the lunch may contain about 8 ounces (230 grams) of fresh vegetables.

The dinner is based primarily upon meat and starch. The meat may be that of a red meat or fish or fowl. Approximately 3 ounces (90 grams) of meat is employed with about 3 ounces (90 grams) of starch.

One of the preferred breakfast, lunch and dinner menus consists of the following -:

WEIGHT LOSS PROGRAM -1000 Calories (4200 Joules)

Breakfast -:

Cereal, ½ cup (All-Bran or Bran Buds)
1 cup skim milk
orange or grapefruit
1-cup of coffee or tea

Lunch -:

Salad (must have 2 carrots and 2 tomatoes, ½ cup beans and/or any other vegetable)
2 lbs. (ca. 900 grams) low calorie dressing or lemon juice
fruit, one piece (apple, pear, peach or two plums)
coffee, tea or diet drink

Dinner -:

one portion of meat *
one portion of starch *
1 -2 portions of vegetable *
fruit, one piece (apple, pear, peach or 2 plums)
coffee, tea or diet drink
(* refer to table of food portions)

FOOD TABLE

Meat Portion (broiled or poached)

3 oz. (90 grams) chicken, one lean pork chop - (3 oz. or 90 grams), one slice roast turkey breast (3 oz. or 90 grams), 4 oz. or 115 grams codfish, ¼ lb - (115 grams) flounder, ½ cup shrimp, 2 eggs

Starch Portion (steamed, boiled, no butter)

1 med. corn on th cob, ½ cup corn, 1 med. baked potato, 1 med. baked or boiled yam, 1 cup whole wheat spinach noddles, 1 cup whole wheat spaghetti (with or without tomato sauce), 1 cup brown rice, 2 slices of bread (seven grain, whole wheat, dark rye, tortillas)

Vegetable Portion (steamed, no butter)

½ cup peas, ¾ cup brocolli, 1 cup green beans, ⅞ cup cauliflower, 2 large slices eggplant, ½ pkg. - (frozen 10 oz. or 285 grams pkg.) peas & carrots, ¾ cup Brussel sprouts, 1 cup cabbage, 1 cup spinach, 1 cup squash.

The composition of the present invention is also highly effective for use in connection with the high protein-low calorie liquid diets or so-called "chemically defined diets". In these diets, generally about five hundred to seven hundred calories (ca 2100 to 3000 joules) per day are received by the patient along with the composition of the present invention. Over a five month period with obese adolesents, 20 to 25 % of the inital weight loss can be obtained and 70 to 75 % of this weigth loss is primarily in fat content. While no significant side effects are noted, there may be some electrocardiographic change.

These low calorie intake diets generally contain the proper amount of L-amino acids, carbohydrates, vitamins and minerals. Generally, the compositions contain about 0.35 grams to about 0.50 grams of amino acid or amino acid mixture per 100 grams of the low calorie diet. Glucose polymers, such as aligasaccharrides, are present in an amount of about 30 grams per 100 grams of the low calorie liquid diet. Salt content, as for example, sodium potassium and calcium salts along with some magnesium salts constitute about 20 grams per 100 grams of the low calorie diet. Vitamins usually are present along with some trace elements, such as potassium idide.

Chemically balanced or low calorie diets are more specifically described in American Journal of Clinical Nutrition, 38: July 1983, pp. 20-31 "A High Protein Low Calorie Liquid Diet In The Treatment of Very Obese Adolescents; Long-Term Effects on Lean Body Mass" by Marilyn R. Brown, M.D. et al; and Metabolism, Volume 23, No. 7 (July) 1974 "Clinical and Metabolic Studies of Chemically Defined Diets In The Management of Obesity" by I. Mc Lean Baird, et al, and the New England Journal of Medicine, March 1, 1984 (editorials) pp. 589-591 "Editorial Retrospective Very Low-Calorie Protein Diets".

The composition of the present invention when used in the amounts as previously described, in combination with the low calorie diet of the type described, results in very substantial weight loss over a relatively confined period of time. Not only is the calorie intake of the patient substantially reduced, but appetite is also satiated.

The invention is further illustrated by, but not limited to the following

EXAMPLES -:

EXAMPLE 1

A composition was formed in accordance with the present invention by mixing 0.25 milligrams of hyoscamine with 5 grams of the guar viscous agent. This composition was administered to a patient having an initial weight of 308 pounds - (139,7 kg) for a six week period. The gastric emptying time at the outset of the six week period was twenty seven minutes. The composition was administered to the patient three times per day. After a four week period, the weight of the individual was 301 pounds (136,5 kg) and after an eight week period the weight of that individual was 291 pounds (132 kg). More importantly, the gastric emptying time had increased to 41 minutes.

EXAMPLE 2

The composition of Example 1 was administered to another patient having an initial weight of 255 pounds (115,7 kg) with a gastric emptying time of ten minutes. The composition was administered three times per day at meals. The weight of the individual after a six week period was 264 pounds (111,6 kg) and the gastric emptying had increased to 57 minutes.

## EXAMPLE 3

A composition of 1.05 milligrams of hyoscamine is mixed with seven grams of the guar compound. This composition is then administered to a patient having an inital emptying time of approximately 27 minutes. After a six week period, the gastric emptying time is about 90 minutes.

## EXAMPLE 4

A composition is formed in accordance with the present invention by mixing 0.27 milligrams of hyoscamine with 5.9 grams of the pectin fiber. The composition is administered to a patient having an initial weight of about 298 pounds (135,2 kg) for a six week period. The gastric emptying time at the outset of the six week period is 29 minutes. The composition is administered to the patient about 3 to 4 times per day. After a four week period, the weight of the individual is down to 288 pounds - (130,6 kg) and after a eight week period, the weight of the individual is down to 276 pounds (125,2 kg). In addition, the gastric emptying time is increased to about 40 minutes.

## EXAMPLE 5

The composition of Example 1 is administered to a patient having an initial weight of 260 pounds - (117,9 kg) with a gastric emptying time of 15 minutes. The composition is administered three times per day at meals along with the dietary program as specified below. The weight of the individual after a six week period is 230 pounds - (104,3 kg) and the gastric emptying time is increased to 60 minutes.

The weight loss program is based upon a dietary plan including -:

WEIGHT LOSS PROGRAM -1000 Calories (ca. 4200 Joules)

Breakfast -:

1 cup cereal (All-Bran)
1 cup skim milk
one orange
coffee

Lunch -:

salad including 2 carrots and 2 tomatoes, ½ cup beans 2 lbs. (ca. 900 grams) lemon juice
fruit, one apple
coffee

Dinner -:

one portion (3 oz. or 90 grams) chicken
one baked potatoe
½ cup peas
one apple
coffee

## EXAMPLE 6

The composition of Example 1 is administered to a patient having an initial weight of 273 pounds - (123,8 kg) with a gastric emptying time of 16 minutes. The composition is administered three times per day along with a low liquid calorie diet program as specified below. The weight of the individual after a six week period is 194 pounds (88 kg) and the gastric emptying time is increased to 60 minutes.

The weight loss program is based upon a low liquid chemical diet plan which includes a daily intake of the following low calorie-high protein liquid diet-:

(1) 15 grams per 100 grams of liquid diet of an amino acid which also contains 0.344 grams of L-isoleucine and 0.408 grams per 100 grams of diet of lysine and 0.367 grams of L-anine per 100 grams of liquid diet;

(2) A glucose polymer of 30 grams per 100 grams of liquid diet which contains about 6 glucose units per unit of polymer;

(3) Twenty grams of a salt mix per 100 grams of liquid diet and which also includes sodium in an amount of about 0.481 grams, potassium in an amount of about 0.440 grams, calcium in an amount of about 0.167 grams, magnesium in an amount of about 35.5 milligrams, phosphate in an amount of about 0.0167 grams and cluconate in an amount of about 1.44 grams; and

(4) 0.5 grams of trace elements which includes about 0.05 grams of potassium iodide and 1.040 grams of copper acetate monohydrate.

In addition to the above, vitamins are supplied by means of a capsule on a daily basis which includes about 5.000 units of vitamin A, 2 milligrams of vitamin B-1 and 2 milligrams of vitamin B-2, 50 milligrams of vitamin C and 400 units of vitamin D. In addition, 1 milliliter of sunflower oil is given daily to supply essential fatty acids.

## Claims

1. A composition to reduce gastric emptying in a selected time frame, said composition comprising:
   a) a major amount of an ingestible fiber compound present in said composition, and
   b) a minor amount of an anticholinergic agent in admixture with said fiber compound such that the composition is highly effective in reducing gastric emptying.

2. The composition of claim 1, characterized in that the ingestible fiber is a polysaccharide containing viscous fiber.

3. The composition of claim 2, characterized in that said viscous fiber compound is comprised of galactose and mannose.

4. The composition of claim 3, characterized in that the viscous fiber is a guar gum.

5. The composition of claim 1, characterized in that the anticholinergic agent has at least 50 %, preferably at least 80 %, bio-availability in the users body.

6. The composition of claim 1, characterized in that the anticholinergic agent is selected from the class consisting of bealladonà agents, antidepresants and antihistimines.

7. The composition of claim 1, characterized in that the anticholinergic agent is selected from the class consisting of atropine, hyoscamine and scopolamine.

8. The composition of claim 1, characterized in that said ingestible fiber is selected from the class consisting of galactomannan fibers, gluco-mannan fibers, pectin fibers, bran fibers, lignin fibers and vegetable cellulose fibers.

9. The composition of claim 1, characterized in that the minor amount of anticholinergic agent ranges from 0.0001 % to about 5.5 %, preferably from 0.0002 to about 3.85 %, based on the total weight of the composition and the viscous fiber makes up the balance of the composition and constitutes the major amount.

10. Use of a composition according to any of the preceding claims for manufacture of a substance for reducing gastric emptying in a selected time frame.

CLAIMS -for AUSTRIA only -:

1. A method for manufacture of a highly effective composition for reducing gastric emptying in a selected time frame, characterized in that a minor amount of an anticholinergic agent is admixed to a major amount of an ingestible fiber compound.

2. Method according to claim 1, characterized in that a polysaccharide containing viscous fiber is used as the ingestible fiber.

3. Method according to claims 1 and 2, characterized in that a viscous fiber compound comprised of galactose and mannose is used.

4. Method according to claim 3, characterized in that the viscous fiber is a guar gum.

5. Method according to any of the preceding claims, characterized in that there is used an anticholinergic agent having at least 50 %, preferably at least 80 %, bio-availability in the users body.

6. Method according to any of the preceding claims, characterized in that the anticholinergic agent is selected from the class consisting of belladona agents, antidepresants and antihistimines.

7. Method according to any of the preceding claims, characterized in that the anticholinergic agent is selected from the class consisting of atropine, hyoscamine and scolopamine.

8. Method according to any of the preceding claims, characterized in that the ingestible fibers are selected from the class consisting of galactomannan fibers, gluco-mannan fibers, pectin fibers, bran fibers, lignin fibers and vegetable cellulose fibers.

9. Method according to any of the claims 2 to 7, characterized in that an amount of anticholinergic agent ranging from 0.0001 % to about 5.5 %, preferably from 0.0002 % to about 3.85 %, based on the total weight of the composition, is admixed to the viscous fiber making up the balance of the composition and constituting its major amount.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 91, no. 15, 8th October 1979, page 512, abstract no. 122560g, Columbus, Ohio, US; S. HOLT et al.: "Effect of gel fiber on gastric emptying and absorption of glucose and paracetamol", & LANCET 1979, 1(8117), 636-9 * Abstract * | 1-10 | A 61 K 31/46 A 23 L 1/308 |
| A | CHEMICAL ABSTRACTS, vol. 90, no. 25, 18th June 1979, page 60, abstract no. 197814f, Columbus, Ohio, US; S.M. CHERNISH et al.: "Comparison of the effects of glucagon and atropine sulfate on gastric emptying", & AM. J. GASTROENTEROL. 1978, 70(6), 581-6 * Abstract * | 1-10 | |

TECHNICAL FIELDS SEARCHED (Int. Cl.4)

A 61 K
A 23 L

The present search report has been drawn up for all claims

| Place of search | Date of completion of the search | Examiner |
|---|---|---|
| THE HAGUE | 11-02-1987 | BRINKMANN C. |